# EUROPEAN PATENT SPECIFICATION

(11) **EP 2 216 039 B1**
(45) Date of publication and mention of the grant of the patent: **18.07.2012**
(21) Application number: 07845761.1
(22) Date of filing: 30.11.2007
(51) Int. Cl.: A61K 36/725, A61K 36/484, A61K 36/258, A61P 25/22

(54) **PHARMACEUTICAL COMPOSITIONS FOR TREATING ANXIETY**
PHARMAZEUTISCHE ZUSAMMENSETZUNGEN ZUR BEHANDLUNG VON ANGSTZUSTÄNDEN
COMPOSITIONS PHARMACEUTIQUES POUR LE TRAITEMENT DE L'ANXIÉTÉ

(43) Date of publication of application: 11.08.2010
(73) Proprietor: Chi, Yu-Fen, Taiwan (CN); Zhang, Zuoguang, Beijing 100000 (CN)
(72) Inventor: ZHANG, Zuoguang, Beijing 100000 (CN)
(74) Representative: Isarpatent
(86) International application number: PCT/CN2007/003398
(87) International publication number: WO 2009/070922

(56) References cited:
- CN-A- 1 836 687
- US-A- 6 083 932
- SHORES M M ET AL: "The relationship between anxiety and depression: A clinical comparison of generalized anxiety disorder, dysthymic disorder, panic disorder, and major depressive disorder", COMPREHENSIVE PSYCHIATRY, GRUNE AND STRATTON, ORLANDO, FL, US, vol. 33, no. 4, 1 July 1992 (1992-07-01), pages 237-244, XP022965650, ISSN: 0010-440X, DOI: DOI:10.1016/0010-440X(92)90047-T [retrieved on 1992-07-01]

## Description

The present invention relates to a pharmaceutical composition or a healthcare food manufactured from the raw materials including ginsenoside Rg1 and Rb1, glycyrrhizic acid and jujuba cyclic adenosine monophosphate (jujuba cAMP) for use in treating anxiety disorder. In particular, the present invention relates to the pharmaceutical composition or the healthcare food for use in treating anxiety disorder; it has definite functions and components, obvious curative effect, fewer side effects and high safety for long-term usage.

Psychological disorders are caused by brain dysfunction and afflicted individuals show abnormalities in awareness, thought, emotion, behavior, will and intelligence, etc. Psychological disorders occupy four in ten diseases, causing the most serious load in society. People are now paying more and more attention to psychological dysfunction in the course of social development. The medical community and the society as a whole are urgently seeking psychological medicine to combat psychological disorders. Anxiety disorder is one common psychological dysfunction, with anti-anxiety pharmaceuticals being the main method of treatment.

Anxiety disorder is a psychological disease manifest mainly as anxious emotion. The main characteristics are breakout or continuous anxious emotions such as anxiety, catatonia and fear, etc., in combination with syndromes such as autonomic nerves disturbance, muscle rigidity and exercise disturbance, etc. Since Sigmund Freud had separated anxiety disorder from neurasthenia, scholars around the world have begun large-scale studies on anxiety disorder and have accumulated a large amount of data. According to modem medical research, the etiology of anxiety disorder includes defects in psychological anatomy, neurotransmitter/modulator-receptor and neuro-endocrine system, etc.

The current mainstream anti-anxiety medicine is benzodiazepine the mechanism of which is to modulate the activity of the inhibitory neurotransmitter, gamma-aminobutyric acid (GABA), to reduce and relieve the symptoms. However, benzodiazepine has many side effects including insomnolence, allergy, muscle pain, weakness, nausea, exercise dysfunction, blurred vision, weariness, disturbance and delusion, etc.

In light of the current situation, the search for a new generation of pharmaceuticals with fewer side effects and more pronounced/potent anti-anxiety qualities has become the center of attention of the entire pharmaceutical world.

It is therefore attempted by the applicant to deal with the above situation encountered in the prior art.

The purpose of the present invention is to provide a pharmaceutical composition or a healthcare food to overcome the insufficiency of the currently available treatments for anxiety disorder. The pharmaceutical composition or a healthcare food is manufactured from the raw materials including ginsenoside Rg1 and Rb1, glycyrrhizic acid and jujuba cAMP for treating anxiety disorder. In particular, the new technical scheme offering definite functions and components, obvious curative effect, fewer side effects and high safety for long-term usage is provided.

The technical scheme of the present medicine is the result endeavored by the inventor. The scheme, employing three raw materials including ginseng, liquorice and jujuba, is developed based on the pathological and pharmacological theories of modem medicine for treating anxiety; specifically, it integrates past pharmaceutical-targeted research with the recent knowledge developed in post-receptor mechanism. Ginsenoside from ginseng has adenylate cyclase (AC) activity to stimulate cAMP synthesis and cAMP phosphodiesterase (CAPD) inhibitory activity to reduce cAMP breakdown; glycyrrhizic acid (and glycyrrhetinic acid) from liquorice are strong inhibitors of CAPD. Ginsenoside Rg1 and Rb1 and glycyrrhizic acid when paired and used collectively further increase the concentration and activity of cAMP and protein kinase A (PKA) respectively in the organism. The increasing concentration and activity of cAMP can catalyze the phosphorylation of GABA α/β subunits and phosphorylation of the β subunit thereof can amplify the inhibitory function of GABA on neurons to achieve significant anti-anxiety functions. Finally, jujuba cAMP from jujube, being the extrinsic non-hydrolyzable cAMP, can participate in the metastasis of cAMP in the organism, stimulating the enzyme function and effectively increasing the expression of cAMP and PKA in the organism, to further enhance the anti-anxiety function. Accordingly, the raw materials including ginsenoside Rg1 and Rb1, glycyrrhizic acid and jujuba cAMP are combined to maximize the effects of anti-anxiety function of the present invention. Ginseng, liquorice and jujuba are commonly used pharmaceutical materials in Chinese medicine and have been used in dietary nourishing medicinal meals for several thousand years. In this long clinical and dietary history, the safety and efficacy of combined use of ginseng, liquorice and jujuba have been sufficiently proven. The inventor's research and experimental results have shown that if these three pharmaceutical materials are only normally decocted and extracted to obtain the extract, the extract does not have significant anti-anxiety effect compared with the mainstream anti-anxiety medicines of the present technology. However, upon further purification of the extract of these three pharmaceutical materials to increase the concentration of the effective components containing ginsenoside Rg1 and Rb1, glycyrrhizic acid and jujuba cAMP, etc., as described in this invention, a pharmaceutical composition with significant anti-anxiety function is obtained. Animal experimental results clearly demonstrate that the manufactured pharmaceutical composition of the present invention has superior anti-anxiety effect compared with the pharmaceutical composition of the mainstream medicine, Diazepam, for treating anxiety disorder. Furthermore, taking ginseng, liquorice and jujuba would not generate side effects as taking the present mainstream medicines for treating anxiety. Patients would not cease or refuse the pharmaceutical therapy for worrying about the side effects. The inventor thus submits that ginsenoside Rg1 and Rb1, glycyrrhizic acid and jujuba cAMP be the raw materials for manufacturing the pharmaceutical composition or the healthcare food for treating anxiety disorder. In particular, the new technical scheme, which has definite functions and components, high safety for long-term usage without side effects, improves the drawbacks generated in the prior art.

Glycyrrhetinic acid has higher liposolubility than glycyrrhizic acid and can easily enter the brain through the blood-brain barrier. Since the glycyrrhizic acid is converted to glycyrrhetinic acid in the human body with almost 100% efficiency, the inhibition of CAPD by glycyrrhizic acid is proceeded by transforming glycyrrhizic acid to glycyrrhetinic acid in the body. Accordingly, glycyrrhizic acid or glycyrrhetinic acid can be the raw material for manufacturing the pharmaceutical composition of the present invention.

In accordance with one aspect of the present invention, a pharmaceutical composition for use in treating anxiety disorder is provided. The pharmaceutical composition includes: ginsenoside having Rg1 and Rb1; a glycyrrhizically related acid being one selected from a group consisting of glycyrrhizic acid, glycyrrhetinic acid and a combination thereof; and jujuba cAMP.

Preferably, the pharmaceutical composition includes 2 ~ 24 parts by weight of ginsenoside, 3 ~ 45 parts by weight of the glycyrrhizically related acid and 0.003 ~ 0.4 parts by weight of jujuba cAMP.

Preferably, the pharmaceutical composition includes 4 ~ 11 parts by weight of ginsenoside, 5 ~ 14 parts by weight of the glycyrrhizically related acid and 0.01 ~ 0.07 parts by weight of jujuba cAMP.

Preferably, ginsenoside is extracted from ginseng, the glycyrrhizically related acid is extracted from liquorice, and jujuba cAMP is extracted from jujuba.

Preferably, jujuba is extracted for obtaining a first extract having a first jujuba cAMP concentration, the first extract is further extracted for obtaining a second extract having a second jujuba concentration, and the second jujuba cAMP concentration is higher than the first jujuba cAMP concentration.

Preferably, the pharmaceutical composition further includes at least one of a pharmacologically acceptable carrier and an additive.

Preferably, the pharmaceutical composition has a dosage form selected from a group consisting of a tablet, a capsule, a powder, a pill, a dust, a solution, a microcapsule, a suspension, an emulsion, a particle, a dropping pill and a roll.

Preferably, the pharmaceutical composition is manufactured as one of a healthcare food and a supplement.

In accordance with another aspect of the present invention, a pharmaceutical composition for use in treating anxiety disorder is provided. The pharmaceutical composition includes: ginsenoside having Rg1 and Rb1; and a glycyrrhizically related acid being one selected from a group consisting of glycyrrhizic acid, glycyrrhetinic acid and a combination thereof.

Preferably, the pharmaceutical composition includes 2 ~ 24 parts by weight of ginsenoside Rg1 and Rb1, and 3 ~ 45 parts by weight of the glycyrrhizically related acid.

Preferably, the pharmaceutical composition includes 4 ~ 11 parts by weight of ginsenoside Rg1 and Rb1, and 5 ~ 14 parts by weight of the glycyrrhizically related acid.

In accordance with another aspect of the present invention, a pharmaceutical composition for use in treating anxiety disorder is provided. The pharmaceutical composition includes ginseng, liquorice and jujuba.

Preferably, the pharmaceutical composition includes 4 ~ 58 parts by weight of ginseng, 2 ~ 28 parts by weight of liquorice and 2 ~ 38 parts by weight of jujuba.

Preferably, the pharmaceutical composition includes 10 ~ 26 parts by weight of ginseng, 5 ~ 13 parts by weight of liquorice and 4 ~ 16 parts by weight of jujuba.

In accordance with another aspect of the present invention, a pharmaceutical composition for use in treating anxiety disorder is provided. The pharmaceutical composition includes ginseng and liquorice.

Preferably, the pharmaceutical composition includes 4 ~ 58 parts by weight of ginseng and 2 ~ 28 parts by weight of liquorice.

Preferably, the pharmaceutical composition includes 10 ~ 26 parts by weight of ginseng and 5 ~ 13 parts by weight of liquorice.

In accordance with another aspect of the present invention, a preparation method of jujuba cAMP of a pharmaceutical composition for use in treating anxiety disorder is provided. The preparation method includes steps of: (a) extracting jujuba for obtaining a first extract having a first jujuba cAMP concentration; and (b) purifying the first extract for obtaining a second extract having a second jujuba cAMP concentration. The second jujuba cAMP concentration is higher than the first jujuba cAMP concentration.

Step (b) is processed by chromatographing the first extract with a macroporous resin bound with an aldehyde group.

Preferably, step (b) further includes steps of: (b1) chromatographing the first extract with an OU-2 macrosporous resin bound with an aldehyde group; and (b2) chromatographing the first extract with an ME-2 macroporous resin bound with the aldehyde group.

The pharmaceutical composition described in the specification and the claims of the present invention for use in treating anxiety disorder is the core content of the present invention. After the present invention is published, one skilled in the art can proceed the normal increase/decrease or substitute for other effective components of the herbal medicine (such as onjisaponin, saikosaponin and liquorice coumarin, etc.) having identical effects/functions to the above-mentioned medicine in accordance with the theory of Chinese medicine or related theory of modem pharmacology. The substitutions of this normal increase/decrease, the herbal medicine having similar mechanism, other identical CAPD inhibitor, AC activator, or corresponding effective components belong to the normal technical activities for one skilled in the art.

The above objectives and advantages of the present invention will become more readily apparent to those ordinarily skilled in the art after reviewing the following detailed descriptions and accompanying drawings, in which:

Fig. 1 is a flowchart showing a preparation method of a pharmaceutical composition in accordance with a first preferred embodiment of the present invention;

Fig. 2 is a flowchart showing a preparation method of a pharmaceutical composition in accordance with a second preferred embodiment of the present invention;

Fig. 3 is a flowchart showing a preparation method of a pharmaceutical composition in accordance with a third preferred embodiment of the present invention;

Fig. 4 is a flowchart showing a preparation method of a pharmaceutical composition in accordance with a fourth preferred embodiment of the present invention;

Fig. 5 is a flowchart showing a preparation method of a pharmaceutical composition in accordance with a fifth preferred embodiment of the present invention; and

Fig. 6 is a flowchart showing a preparation method of a pharmaceutical composition in accordance with a sixth preferred embodiment of the present invention.

The present invention will now be described more specifically with reference to the following Embodiments. It is to be noted that the following descriptions of preferred Embodiments of this invention are presented herein for purpose of illustration and description only; it is not intended to be exhaustive or to be limited to the precise form disclosed.

In order to accomplish the purpose of the present invention, the technical schemes of the present invention are particularly provided as follows.

### Example 1:

The pharmaceutical composition of the present invention for treating anxiety disorder is manufactured from the raw materials of ginseng and liquorcie.

### Example 2:

The pharmaceutical composition of the present invention for treating anxiety disorder is manufactured from the raw materials having 4 ~ 58 parts by weight of ginseng and 2 ~ 28 parts by weight of liquorice.

### Example 3:

The pharmaceutical composition of the present invention for treating anxiety disorder is manufactured from the raw materials having 10 ~ 26 parts by weight of ginseng and 5 ~ 13 parts by weight of liquorice.

### Example 4:

The pharmaceutical composition of the present invention for treating anxiety disorder is manufactured from the raw materials of ginseng, liquorice and jujuba.

### Example 5:

The pharmaceutical composition of the present invention for treating anxiety disorder is manufactured from the raw materials having 4 ~ 58 parts by weight of ginseng, 2 ~ 28 parts by weight of liquorice and 2 ~ 38 parts by weight of jujuba.

### Example 6:

The pharmaceutical composition of the present invention for treating anxiety disorder is manufactured from the raw materials having 10 ~ 26 parts by weight of ginseng, 5 ~ 13 parts by weight of liquorice and 4 ~ 16 parts by weight of jujuba.

### Example 7:

The pharmaceutical composition of the present invention for treating anxiety disorder is manufactured from the raw materials of ginsenoside Rg 1 and Rb1, and glycyrrhizic acid (or glycyrrhetinic acid).

### Example 8:

The pharmaceutical composition of the present invention for treating anxiety disorder is manufactured from 2 ~ 24 parts by weight of ginsenoside Rg1 and Rb1, and 3 ~ 45 parts by weight of glycyrrhizic acid (or glycyrrhetinic acid).

### Example 9:

The pharmaceutical composition of the present invention for treating anxiety disorder is manufactured from 4 ~ 11 parts by weight of ginsenoside Rg1 and Rb1, and 5 ~ 14 parts by weight of glycyrrhizic acid (or glycyrrhetinic acid).

### Example 10:

The pharmaceutical composition of the present invention for treating anxiety disorder is manufactured from the raw materials of the ginseng extract having the above-mentioned parts by weight of ginsenoside Rg1 and Rb1, and the liquorice extract having the above-mentioned parts by weight of glycyrrhizic acid.

### Example 11:

The pharmaceutical composition of the present invention for treating anxiety disorder is manufactured from the raw materials of ginsenoside Rg1 and Rb1, glycyrrhizic acid (or glycyrrhetinic acid) and jujuba cAMP.

### Example 12:

The pharmaceutical composition of the present invention for treating anxiety disorder is manufactured from the raw materials having 2 ~ 24 parts by weight of ginsenoside Rg1 and Rb1, 3 ~ 45 parts by weight of glycyrrhizic acid (or glycyrrhetinic acid) and 0.003 ~ 0.4 parts by weight of jujuba cAMP.

### Example 13:

The pharmaceutical composition of the present invention for treating anxiety disorder is manufactured from the raw materials having 4 ~ 11 parts by weight of ginsenoside Rg1 and Rb1, 5 ~ 14 parts by weight of glycyrrhizic acid (or glycyrrhetinic acid) and 0.01 ~ 0.07 parts by weight of jujuba cAMP.

### Example 14:

The pharmaceutical composition of the present invention for treating anxiety disorder is manufactured from the raw materials of the ginseng extract having the above-mentioned parts by weight of ginsenoside Rg1 and Rb1, the liquorice extract having the above-mentioned parts by weight of glycyrrhizic acid, and the jujuba extract having the above-mentioned parts by weight of jujuba cAMP.

### Example 15:

The pharmaceutical composition of the present invention is provided, wherein the raw material having jujuba cAMP is the second extract described as follows. First, jujuba is extracted for obtaining the first extract, then the first extract is further extracted for obtaining the second extract, wherein the jujuba cAMP concentration of the second extract is higher than that of the first extract.

### Example 16:

The preparation method of the pharmaceutical composition including the raw material of jujuba cAMP is provided. The preparation method thereof includes the steps as follows.

(a) Jujuba is extracted for obtaining a first extract; and

(b) the first extract is further purified for obtaining a second extract, and the jujuba cAMP concentration of the second extract is higher than that of the first extract.

### Example 17:

In the aforementioned preparation method, the step (b) is processed by chromatographing, absorbing and separating jujuba cAMP of the first extract with the macroporous resin bound with the aldehyde group.

### Example 18:

In the aforementioned preparation method, the step (b) is processed by chromatographing, absorbing and separating jujuba cAMP of the first extract with the OU-2 macroporous resin bound with the aldehyde group.

### Example 19:

In the aforementioned preparation method, the step (b) is processed by chromatographing, absorbing and separating jujuba cAMP of the first extract with the ME-2 macroporous resin bound with the aldehyde group.

### Example 20:

The pharmaceutical composition of the present invention includes the pharmacologically acceptable carriers or additives.

### Example 21:

The pharmaceutical composition of the present invention can be manufactured as a dosage form, and the dosage forms is selected from any one of a tablet, a capsule, a powder, a pill, a dust, a solution, a microcapsule, a suspension, an emulsion, a particle, a dropping pill, a roll and the pharmacologically oral pharmaceutical dosage form.

### Example 22:

The pharmaceutical composition of the present invention further can be manufactured as healthcare food and nutrient supplements.

In order to accomplish the purpose of the present invention, the preparation methods of the pharmaceutical composition are provided as follows.

### Method 1:

The pharmaceutical composition of the present invention for treating anxiety disorder is manufactured from the extract having ginsenoside Rg1 and Rb1 and glycyrrhizic acid, wherein the extract is extracted and purified from the raw materials having 4 ~ 58 parts by weight of ginseng and 2 ~ 28 parts by weight of liquorice.

### Method 2:

The pharmaceutical composition of the present invention for treating anxiety disorder is manufactured from the extract having ginsenoside Rg1 and Rb1 and glycyrrhizic acid, wherein the extract is extracted and purified from the raw materials having 10 ~ 26 parts by weight of ginseng and 5 ~ 13 parts by weight of liquorice.

### Method 3:

The pharmaceutical composition of the present invention for treating anxiety disorder is manufactured from the extract having ginsenoside Rg1 and Rb1, glycyrrhizic acid and jujuba cAMP, wherein the extract is extracted and purified from the raw materials having 4 ~ 58 parts by weight of ginseng, 2 ~ 28 parts by weight of liquorice and 2 ~ 38 parts by weight of jujuba.

### Method 4:

The pharmaceutical composition of the present invention for treating anxiety disorder is manufactured from the extract having ginsenoside Rg1 and Rb1, glycyrrhizic acid and jujuba cAMP, wherein the extract is extracted and purified from the raw materials having 10 ~ 26 parts by weight of ginseng, 5 ~αβ 13 parts by weight of liquorice and 4 ~ 16 parts by weight of jujuba.

### Method 5:

The pharmaceutical composition of the present invention for treating anxiety disorder is manufactured from (1) the extracts having ginsenoside Rg 1 and Rb1 extracted and purified from ginseng, and glycyrrhizic acid extracted and purified from liquorice; or (2) the prepared raw materials having ginsenoside Rg1 and Rb1, and glycyrrhizic acid (or glycyrrhetinic acid).

### Method 6:

The pharmaceutical composition of the present invention for treating anxiety disorder is manufactured from the raw materials having 2 ~ 24 parts by weight of ginsenoside Rg1 an Rb1, and 3 ~αβ 45 parts by weight of glycyrrhizic acid (or glycyrrhetinic acid).

### Method 7:

The pharmaceutical composition of the present invention for treating anxiety disorder is manufactured from the raw materials having 4 ~ 11 parts by weight of ginsenoside Rg1 an Rb1, and 5 ~ 14 parts by weight of glycyrrhizic acid (or glycyrrhetinic acid).

### Method 8:

The pharmaceutical composition of the present invention for treating anxiety disorder is manufactured from (1) the extracts having ginsenoside Rg1 and Rb1, glycyrrhizic acid and jujuba cAMP respectively extracted and purified from ginseng, liquorice and jujuba; or (2) the prepared raw materials having ginsenoside Rg1 and Rb1, glycyrrhizic acid (or glycyrrhetinic acid) and jujuba cAMP.

### Method 9:

The pharmaceutical composition of the present invention for treating anxiety disorder is manufactured from the raw materials having 2 ~ 24 parts by weight of ginsenoside Rg1 an Rb1, 3 ~ 45 parts by weight of glycyrrhizic acid (or glycyrrhetinic acid) and 0.003 - 0.4 parts by weight of jujuba cAMP.

### Method 10:

The pharmaceutical composition of the present invention for treating anxiety disorder is manufactured from the raw materials having 4 ~ 11 parts by weight of ginsenoside Rg1 an Rb1, 5 ~ 14 parts by weight of glycyrrhizic acid (or glycyrrhetinic acid) and 0.01 ~ 0.07 parts by weight of jujuba cAMP.

### Method 11:

The preparation method of the pharmaceutical composition including the raw material of jujuba cAMP is provided. The preparation method thereof includes the steps as follows.

(a) Jujuba is extracted for obtaining a first extract; and

(b) the first extract is further purified for obtaining a second extract, and the jujuba cAMP concentration of the second extract is higher than that of the first extract.

### Method 12:

In the aforementioned preparation method, the step (b) is processed by chromatographing, absorbing and separating jujuba cAMP of the first extract with the macroporous resin bound with the aldehyde group.

### Method 13:

In the aforementioned preparation method, the step (b) is processed by chromatographing, absorbing and separating jujuba cAMP of the first extract with the OU-2 macroporous resin bound with the aldehyde group.

### Method 14:

In the aforementioned preparation method, the step (b) is processed by chromatographing, absorbing and separating jujuba cAMP of the first extract with the ME-2 macroporous resin bound with the aldehyde group.

### Method 15:

The pharmaceutical composition of the present invention includes the pharmacologically acceptable carriers or additives.

### Method 16:

The pharmaceutical composition of the present invention can be manufactured as a dosage form, and the dosage forms is selected from any one of a tablet, a capsule, a powder, a pill, a dust, a solution, a microcapsule, a suspension, an emulsion, a particle, a dropping pill, a roll and the pharmacologically oral pharmaceutical dosage form.

### Method 17:

The raw materials described in the present invention can be manufactured as the medicines, healthcare food and nutrient supplements for treating anxiety disorder in accordance with the Good Manufacturing Practice (GMP) pharmaceutical standards and the method of healthcare food producing/manufacturing standards.

### THE PREPERRED EMBODIMENT

The present invention is further illustrated as follows by combining the figures and the preferred embodiments.

### Embodiment 1:

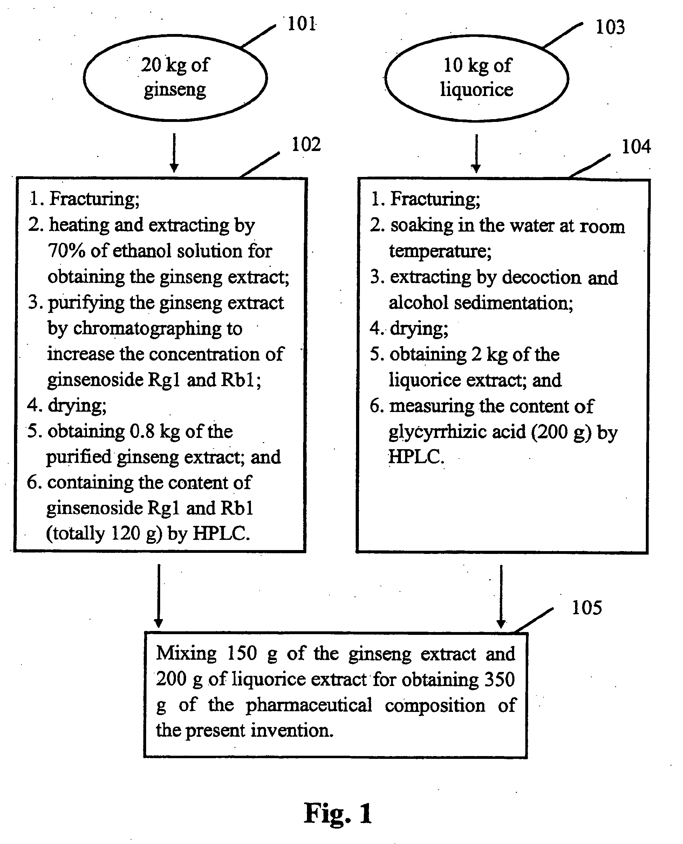

Please refer to Fig. 1, which is the flowchart showing a preparation method of a pharmaceutical composition in accordance with a first preferred embodiment of the present invention. In Fig. 1, after 20 kg of ginseng (101) is fractured, the fractured ginseng is heated to extract by 70% of the ethanol solution. The extracted ginseng is separated and purified by chromatography, and dried, and 0.8 kg of the ginseng extract having 120 g of ginsenoside Rg1 and Rb1 is obtained (102). Further, after 10 kg of liquorice (103) is fractured, the fractured liquorice is soaked at room temperature for 12 hours. The soaked liquorice is extracted by decoction and alcohol sedimentation, concentrated and dried, and 2 kg of the liquorice extract having 200 g of glycyrrhizic acid is obtained (104). Next, 150 g of the obtained ginseng extract and 200 g of the obtained liquorice extract are pulverized and mixed, and 350 g of the pharmaceutical composition (containing 22.5 g of ginsenoside Rg1 and Rb1, and 20 g of glycyrrhizic acid) of the present invention is obtained (105).

### Embodiment 2:

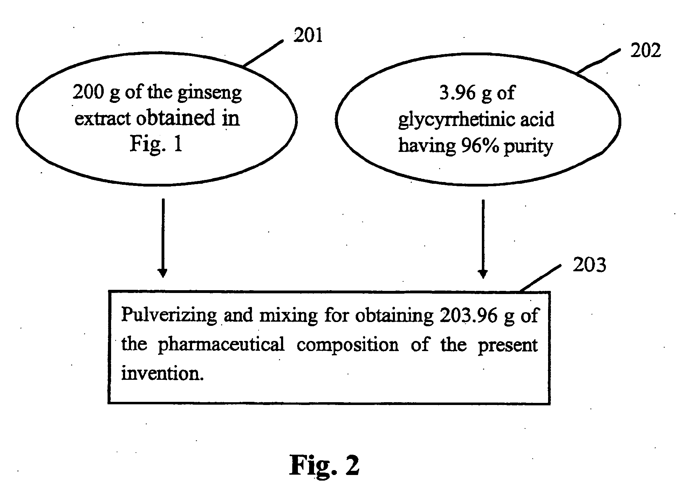

Please refer to Fig. 2, which is the flowchart showing a preparation method of a pharmaceutical composition in accordance with a second preferred embodiment of the present invention. In Fig. 2, after the prepared 3.96 g of glycyrrhetinic acid having 96% purity (202) and 200 g of the ginseng extract obtained in Embodiment 1 (201) are pulverized and mixed, 203.96 g of the pharmaceutical composition (containing 30 g of ginsenoside Rg1 and Rb1, and 3.8 g of glycyrrhetinic acid) of the present invention is obtained (203).

### Embodiment 3:

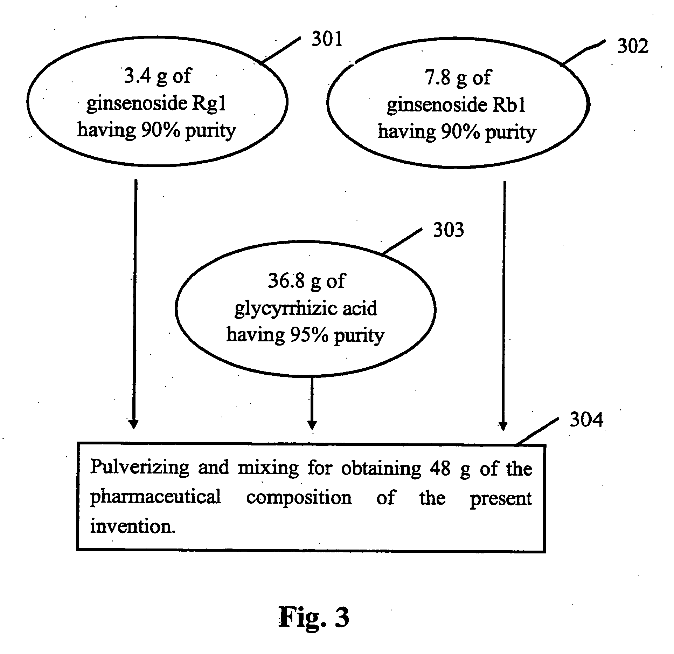

Please refer to Fig. 3, which is the flowchart showing a preparation method of a pharmaceutical composition in accordance with a third preferred embodiment of the present invention. In Fig. 3, after 3.4 g of the prepared ginsenoside Rg1 having 90% purity (301), 7.8 g of the prepared gensenoside Rb1 having 90% purity (302) and 36.8 g of glycyrrhizic acid having 90% purity (303) are pulverized and mixed, 48 g of the pharmaceutical composition (containing 10 g of ginsenoside Rg1 and Rb1, and 35 g of glycyrrhizic acid) of the present invention is obtained (304).

### Embodiment 4:

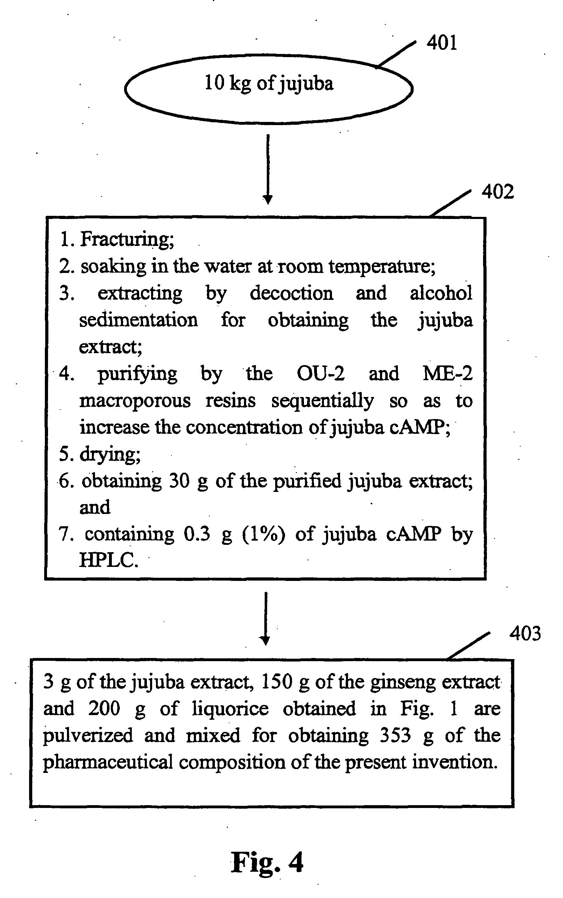

Please refer to Fig. 4, which is the flowchart showing a preparation method of a pharmaceutical composition in accordance with a fourth preferred embodiment of the present invention. In Fig. 4, 10 kg of jujuba (401) is fractured and soaked in the water at room temperature, then the soaked jujuba is extracted by decoction and alcohol sedimentation for obtaining the jujuba extract, which is further absorbed and separated by the OU-2 and ME-2 macroporous resins sequentially, and dried. Thirty (30) g of the jujuba extract containing 0.3 g of jujuba cAMP is obtained to be the raw material for preparing the medicine of the present invention (402).

Afterwards, after 150 g of the ginseng extract and 200 g of the liquorice extract obtained in Embodiment 1 are pulverized and mixed with 3 g of the above-mentioned jujuba extract, and 353 g of the pharmaceutical composition (containing 22.5 g of ginsenoside Rg1 and Rb1, 20 g of glycyrrhizic acid and 0.03 g of jujuba cAMP) of the present invention is obtained (403).

### Embodiment 5:

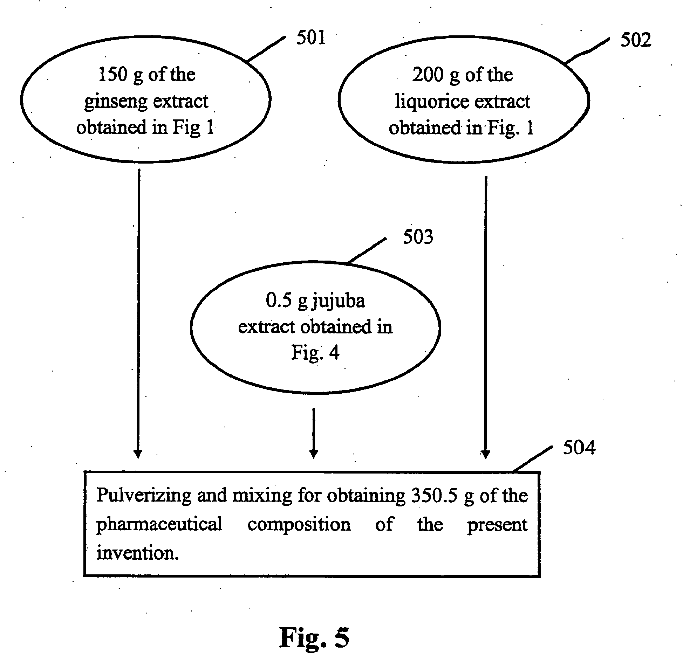

Please refer to Fig. 5, which is the flowchart showing a preparation method of a pharmaceutical composition in accordance with a fifth preferred embodiment of the present invention. In Fig. 5, after 150 g of the ginseng extract (501) and 200 g of the liquorice extract (502) obtained in Embodiment 1 respectively are pulverized and mixed with 0.5 g of the jujuba extract obtained in Embodiment 4 (503), 350.5 g of the pharmaceutical composition (containing 22.5 g of ginsenoside Rg1 and Rb1, 20g of glycyrrhizic acid and 0.005 g of jujuba cAMP) of the present invention is obtained (504).

### Embodiment 6:

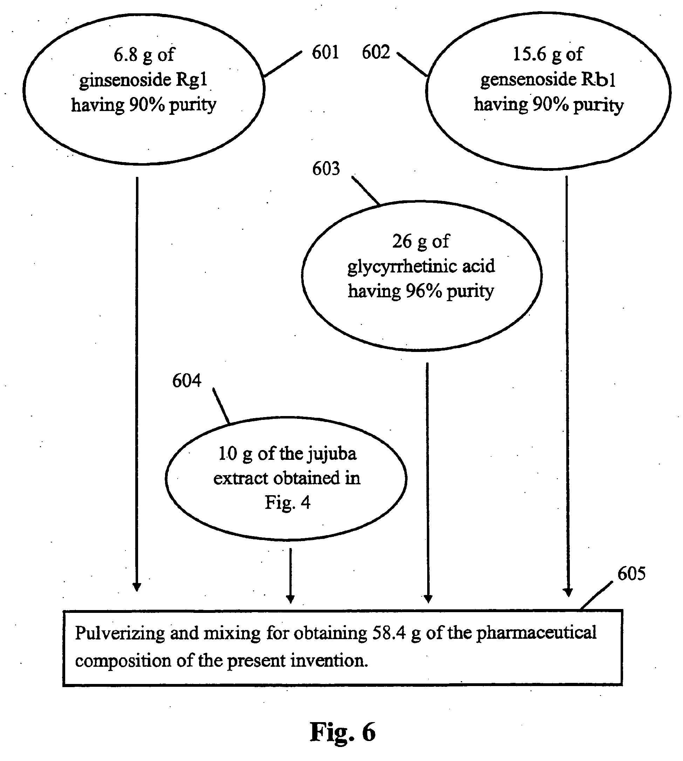

Please refer to Fig. 6, which is the flowchart showing a preparation method of a pharmaceutical composition in accordance with a sixth preferred embodiment of the present invention. In Fig. 6, after 6.8 g of the prepared ginsenoside Rg1 having 90% purity (601), 15.6 g of the prepared ginsenoside Rb1 having 90% purity (602), 26 g of glycyrrhetinic acid having 96% purity (603) and 10 g of the jujuba extract obtained in Embodiment 4 (604) are pulverized and mixed, 58.4 g of the pharmaceutical composition (containing 20 g of ginsenoside Rg1 and Rb1, 25 g of glycyrrhetinic acid and 0.1 g of jujuba cAMP) of the present invention is obtained (605).

### Experiment 1: The influence of Embodiment 1 in the light-dark transition experiment of the mouse

1.1 Experimental animals: Kunming (KM) mice, male, 24 ~ 26 g of body weight, secondary, are provided by the Experimental Animal Science Department of Capital Medical University, Beijing.

1.2 Experimental pharmaceuticals: The pharmaceutical of Embodiment 1 is provided by Beijing Wonner Biotech. Ltd. Co., and Diazepam is the product of Tianjin Jinhuei Amino Acid Co. Ltd.

1.3 Experimental equipment: Self-made light-dark transition box.

1.4 Dose designs: 1. High dose of Embodiment 4 (80 mg/kg/d); 2. middle dose of Embodiment 4 (40 mg/kg/d); and 3. low dose of Embodiment 4 (20 mg/kg/d).

1.5 Experimental method and result:

1.5.1 Group division and administration of drug: The mice are grouped randomly as 5 groups, and 10 mice are in each group. 1. High dose of Embodiment 1 (80 mg/kg); 2. middle dose of Embodiment 1 (40 mg/kg); 3. low dose of Embodiment 1 (20 mg/kg); 4. Diazepam (2.5 mg/kg); and 5. physiological saline (normal saline, NS). The drugs are fed into the mouse stomach once every day, and the mouse is administered for continuous 7 days. During the period of administration, the mouse eats and drinks freely, and the experiment is proceeded after 1 hour of the last administration of drug on the eighth day.

1.5.2 Experimental method:

Mouse light-dark transition test: The dark chamber occupies one third of the light-dark transition chamber (44 cm x 21 cm x 21 cm), and the top is capped. The light chamber occupies two third thereof and is illuminated brightly. A door between two chambers is disposed for the passage of the mouse. The mouse is placed in the center of the light chamber when the experiment begins, and the mouse's back faces the dark chamber. The times that the mouse enters into the dark chamber and returns to the light chamber within 10 minutes are determined, and the times thereof are the index for evaluating the anti-anxiety function of the drugs.

1.5.3 Statistic calculation: The experimental data are represented as *X̅*±*SD*, and the experimental result is calculated as one-way analysis of variance (one-way ANOVA) by SPSS 11.5 statistic software.

1.5.4 Experimental result: Please refer to Table 1.

**Table 1. The influence of Embodiment 1 on the times of the mouse light-dark transition experiment**

| Group | Animal number | Times of passing from dark chamber to light chamber |
|---|---|---|
| High dose of Embodiment 1 | 10 | 11.2±3.84* |
| Middle dose of Embodiment 1 | 10 | 13.1±5.38** |
| Low dose of Embodiment 1 | 10 | 13.5±4.65** |
| Diazepam | 10 | 11.3±4.54* |
| Normal saline (NS) | 10 | 6.2±4.32 |

| | | |
|---|---|---|
| In comparison with the NS group: *P < 0.05, and **P < 0.01. | | |

1.6 Description: The light-dark transition experiment adopted in the present experiment is built up on the basis that the mouse congenitally hates the bright light and the voluntary exploring behavior to the new environment. The clinical medicine (Diazepam) for treating anxiety in human beings and the Embodiment 1 have excellent correlation on improving the function of the increasing voluntary exploring behavior on this mouse model. According to the above experiment, it can be found that the high, middle and low doses of Embodiment 1 of the present invention and Diazepam all significantly increase the times that the mouse passes from the dark chamber to the light chamber, and have statistical meanings while comparing with the physiological saline. The experimental result has proven that the Embodiment 1 has anti-anxiety effect.

1.7 Conclusion: According to the above experimental result, it shows that the high, middle and low doses of Embodiment 1 of the present invention and Diazepam all significantly increase the times that the mouse passes from the dark chamber to the light chamber. The result shows the Embodiment 1 having anti-anxiety effect.

### Experiment 2: The influence of Embodiment 4 in the mouse light-dark transition experiment

2.1 Experimental animals: Kunming (KM) mice, male, 24 ~ 26 g of body weight, secondary, are provided by the Experimental Animal Science Department of Capital Medical University, Beijing.

2.2 Experimental pharmaceuticals: The pharmaceutical of Embodiment 4 is provided by Beijing Wonner Biotech. Ltd. Co., and Diazepam is the product of Tianjin Jinhuei Amino Acid Co. Ltd.

2.3 Experimental equipment: Self-made light-dark transition box.

2.4 Dose designs: 1. High dose of Embodiment 4 (80 mg/kg/d); 2. middle dose of Embodiment 4 (40 mg/kg/d); and 3. low dose of Embodiment 4 (20 mg/kg/d).

2.5 Experimental method and result:

2.5.1 Group division and administration of drug: The mice are grouped randomly as 5 groups, and 10 mice are in each group. 1. High dose of Embodiment 4 (80 mg/kg); 2. middle dose of Embodiment 4 (40 mg/kg); 3. low dose of Embodiment 4 (20 mg/kg); 4. Diazepam (2.5 mg/kg); and 5. physiological saline (normal saline, NS). The drugs are fed into the mouse stomach once every day, and the mouse is administered for continuous 7 days. During the period of administration, the mouse eats and drinks freely, and the experiment is proceeded after 1 hour of the last administration of drug on the eighth day.

2.5.2 Experimental method:

Mouse light-dark transition test: The dark chamber occupies one third of the light-dark transition chamber (44 cm × 21 cm × 21 cm), and the top is capped. The light chamber occupies two third thereof and is illuminated brightly. A door between two chambers is disposed for the passage of the mouse. The mouse is placed in the center of the light chamber when the experiment begins, and the mouse's back faces the dark chamber. The times that the mouse enters into the dark chamber and returns to the light chamber within 10 minutes are determined, and the times thereof are the index for evaluating the anti-anxiety function of the drugs.

2.5.3 Statistic calculation: The experimental data are represented as *X̅*±*SD*, and the experimental result is calculated as one-way ANOVA by SPSS 11.5 statistic software.

2.5.4 Experimental result: Please refer to Table 2.

**Table 2. The influence of Embodiment 4 on the times of the mouse light-dark transition experiment**

| Group | Animal number | Times of passing from dark chamber to light chamber |
|---|---|---|
| High dose of Embodiment 4 | 10 | 11.6±2.53* |
| Middle dose of Embodiment 4 | 10 | 13.9±3.76** |
| Low dose of Embodiment 4 | 10 | 13.4±4.12** |
| Diazepam | 10 | 11.7±4.47* |
| Normal saline (NS) | 10 | 6.8±3.85 |

| | | |
|---|---|---|
| In comparison with the NS group: *P < 0.05, and **P < 0.01. | | |

2.6 Description: The light-dark transition experiment adopted in the present experiment is built on the basis that the mouse congenitally hates the bright light and the voluntary exploring behavior to the new environment. The clinical pharmaceutical (Diazepam) for treating anxiety in human beings and the Embodiment 4 have excellent correlation on improving the function of the increasing voluntary exploring behavior of the mouse on this model. According to the above experiment, it can be found that the high, middle and low doses of Embodiment 4 of the present invention and Diazepam all significantly increase the times that the mouse passes from the dark chamber to the light chamber, and have statistical meanings while comparing with the normal saline. The experimental result has proven that the Embodiment 4 has anti-anxiety effect.

2.7 Conclusion: According to the above experimental result, the high, middle and low doses of Embodiment 4 of the present invention and Diazepam all significantly increase the times that the mouse passes from the dark chamber to the light chamber. The result demonstrates the Embodiment 4 has anti-anxiety function.

### Industrial usefulness:

The application scope of the pharmaceutical composition for use according to the present invention for treating anxiety disorder lies in that:

1. the described pharmaceutical composition for use according to the present invention for treating anxiety disorder can include the pharmacologically acceptable additives;

2. the described pharmaceutical composition for use according to the present invention for treating anxiety disorder can be manufactured as the known dosage forms, such as powder, capsule, tablet, etc.; and

3. the described pharmaceutical composition for use according to the present invention for treating anxiety disorder can be manufactured as the healthcare food for treating depression.

## Claims

1. A pharmaceutical composition for use in the treatment of anxiety disorder, **characterized by** comprising a ginseng and a liquorice.

2. The pharmaceutical composition for use according to claim 1 **characterized by** comprising 4 ~ 58 parts by weight of the ginseng and 2 ~ 28 parts by weight of the liquorice, and preferably **characterized by** comprising 10 ~ 26 parts by weight of the ginseng and 5 ~ 13 parts by weight of the liquorice.

3. The pharmaceutical composition for use according to claim 1 further **characterized by** comprising a jujuba.

4. The pharmaceutical composition for use according to claim 3 **characterized by** comprising 4 ~ 58 parts by weight of the ginseng, 2 ~ 28 parts by weight of the liquorice and 2 ~ 38 parts by weight of the jujuba, and preferably **characterized by** comprising 10 ~ 26 parts by weight of the ginseng, 5 ~ 13 parts by weight of the liquorice and 4 ~ 16 parts by weight of the jujuba.

5. The pharmaceutical composition for use according to claim 3, **characterized in that** the jujuba is extracted for obtaining a first extract having a first jujuba cAMP concentration, the first extract is further extracted for obtaining a second extract having a second jujuba concentration, and the second jujuba cAMP concentration is higher than the first jujuba cAMP concentration.

6. A pharmaceutical composition for use in the treatment of anxiety disorder, **characterized by** comprising:
a ginsenoside having an Rg1 and an Rb1; and
a glycyrrhizically related acid being one selected from a group consisting of a glycyrrhizic acid, a glycyrrhetinic acid and a combination thereof.

7. The pharmaceutical composition for use according to claim6 **characterized by** comprising z 24 parts by weight of the ginsenoside and 3 ~ 45 parts by weight of the glycyrrhizically related acid, and preferably **characterized by** comprising 4 ~ 11 parts by weight of the ginsenoside and 5 ~ 14 parts by weight of the glycyrrhizically related acid.

8. The pharmaceutical composition for use according to claim 6 further **characterized by** comprising a jujuba cyclic adenosine monophosphate (jujuba cAMP).

9. The pharmaceutical composition for use according to claim8 **characterized by** comprising 2 ~ 24 parts by weight of the ginsenoside, 3 ~ 45 parts by weight of the glycyrrhizically related acid and 0.003 ~ 0.4 parts by weight of the jujuba cAMP, and preferably **characterized by** comprising 4 ~ 11 parts by weight of the ginsenoside, 5 ~ 14 parts by weight of the glycyrrhizically related acid and 0.01 ~ 0.07 parts by weight of the jujuba cAMP.

10. The pharmaceutical composition for use according to claim 6, **characterized in that** the ginsenoside is extracted from a ginseng, the glycyrrhizically related acid is extracted from a liquorice, and the jujuba cAMP is extracted from a jujuba.

11. The pharmaceutical composition for use according to claim 6 further **characterized by** comprising at least one of a pharmacologically acceptable carrier and an additive, being manufactured as one of a healthcare food and a supplement, and having a dosage form selected from a group consisting of a tablet, a capsule, a powder, a pill, a dust, a solution, a microcapsule, a suspension, an emulsion, a particle, a dropping pill and a roll.

12. A preparation method of a jujuba cyclic adenosine monophosphate (jujuba cAMP) of a pharmaceutical composition for treating an anxiety disorder, **characterized by** comprising steps of:
(a) extracting a jujuba for obtaining a first extract having a first jujuba cAMP concentration; and
(b) purifying the first extract by chromatographing the first extract with a macroporous resin bound with an aldehyde group for obtaining a second extract having a second jujuba cAMP concentration,
wherein the second jujuba cAMP concentration is higher than the first jujuba cAMP concentration.

## Patentansprüche

1. Pharmazeutische Zusammensetzung zur Verwendung bei der Behandlung von krankhaften Angstzuständen, **dadurch gekennzeichnet**, 1 dass sie einen Ginseng und ein Süßholz enthält.

2. Pharmazeutische Zusammensetzung zur Verwendung nach Anspruch 1, **dadurch gekennzeichnet**, 1 dass sie 4 bis 58 Gewichtsteile des Ginseng und 2 bis 28 Gewichtsteile des Süßholzes enthält, und bevorzugt **dadurch gekennzeichnet, dass** sie 10 bis 26 Gewichtsteile des Ginseng und 5 bis 13 Gewichtsteile des Süßholzes enthält.

3. Pharmazeutische Zusammensetzung zur Verwendung nach Anspruch 1, des Weiteren **dadurch gekennzeichnet**, 1 dass sie eine Jujube enthält.

4. Pharmazeutische Zusammensetzung zur Verwendung nach Anspruch 3, **dadurch gekennzeichnet, dass** sie 4 bis 58 Gewichtsteile des Ginseng, 1 2 bis 28 Gewichtsteile des Süßholzes und 2 bis 38 Gewichtsteile der Jujube enthält, 1 und bevorzugt **dadurch gekennzeichnet**, 1 dass sie 10 bis 26 Gewichtsteile des Ginseng, 5 bis 13 Gewichtsteile des Süßholzes und 4 bis 16 Gewichtsteile der Jujube enthält.

5. Pharmazeutische Zusammensetzung zur Verwendung nach Anspruch 3, **dadurch gekennzeichnet, dass** die Jujube extrahiert wird, um einen ersten Extrakt mit einer ersten Jujube-cAMP-Konzentration zu erhalten, der erste Extrakt weiter extrahiert wird, um einen zweiten Extrakt mit einer zweiten Jujube-Konzentration zu erhalten, und die zweite Jujube-cAMP-Konzentration höher ist als die erste Jujube-cAMP-Konzentration.

6. Pharmazeutische Zusammensetzung zur Verwendung bei der Behandlung von krankhaften Angstzuständen, **dadurch gekennzeichnet, dass** sie Folgendes enthält:
ein Ginsenosid mit einem Rg1 und einem Rb1; und
eine glycyrrhinisch verwandte Säure, die unter einer Glycyrrhinsäure, einer Glycyrrhetinsäure und einer Kombination davon ausgewählt ist.

7. Pharmazeutische Zusammensetzung zur Verwendung nach Anspruch 6, **dadurch gekennzeichnet, dass** sie 2 bis 24 Gewichtsteile des Ginsenosids und 3 bis 45 Gewichtsteile der glycyrrhinisch verwandten Säure enthält, und bevorzugt **dadurch gekennzeichnet, dass** sie 4 bis 11 Gewichtsteile des Ginsenosids und 5 bis 14 Gewichtsteile der glycyrrhinisch verwandten Säure enthält.

8. Pharmazeutische Zusammensetzung zur Verwendung nach Anspruch 6, des Weiteren **dadurch gekennzeichnet, dass** sie ein zyklisches Jujube-Adenosinmonophosphat (Jujube-cAMP) enthält.

9. Pharmazeutische Zusammensetzung zur Verwendung nach Anspruch 8, **dadurch gekennzeichnet, dass** sie 2 bis 24 Gewichtsteile des Ginsenosids, 3 bis 45 Gewichtsteile der glycyrrhinisch verwandten Säure und 0,003 bis 0,4 Gewichtsteile des Jujube-cAMP enthält, und bevorzugt **dadurch gekennzeichnet, dass** sie 4 bis 11 Gewichtsteile des Ginsenosids, 5 bis 14 Gewichtsteile der glycyrrhinisch verwandten Säure und 0,01 bis 0,07 Gewichtsteile des Jujube-cAMP enthält.

10. Pharmazeutische Zusammensetzung zur Verwendung nach Anspruch 6, **dadurch gekennzeichnet, dass** das Ginsenosid aus einem Ginseng extrahiert wird, die glycyrrhinisch verwandte Säure aus einem Süßholz extrahiert wird und das Jujube-cAMP aus einem Jujube extrahiert wird.

11. Pharmazeutische Zusammensetzung zur Verwendung nach Anspruch 6, des Weiteren **dadurch gekennzeichnet, dass** sie einen pharmakologisch unbedenklichen Träger und/oder einen Zusatzstoff enthält, als ein Gesundheitslebensmittel oder ein Nahrungsergänzungsmittel hergestellt wird und eine Darreichungsform hat, die unter Folgendem ausgewählt ist: Tablette, Kapsel, grobes Pulver, Pille, feines Pulver, Lösung, Mikrokapsel, Suspension, Emulsion, Partikel, Tropfen und Rolle.

12. Zubereitungsverfahren für ein zyklisches Jujube-Adenosinmonophosphat (Jujube-cAMP) einer pharmazeutischen Zusammensetzung zur Verwendung bei der Behandlung von krankhaften Angstzuständen, **dadurch gekennzeichnet, dass** es Folgende Schritte umfasst:
(a) Extrahieren einer Jujube, um einen ersten Extrakt mit einer ersten Jujube-cAMP-Konzentration zu erhalten; und
(b) Reinigen des erstes Extrakts durch Chromatografieren des erstes Extrakts mit einem makroporösen Harz, das mit einer Aldehydgruppe gebunden ist, um einen zweiten Extrakt mit einer zweiten Jujube-cAMP-Konzentration zu erhalten,
wobei die zweite Jujube-cAMP-Konzentration höher ist als die erste Jujube-cAMP-Konzentration.

## Revendications

1. Composition pharmaceutique à utiliser pour traiter des troubles d'anxiété, **caractérisée en ce qu'**elle comprend du ginseng et de la réglisse.

2. Composition pharmaceutique à utiliser selon la revendication 1, **caractérisée en ce qu'**elle comprend de 4 à 58 parties en poids de ginseng et de 2 à 28 parties en poids de réglisse, de préférence caractérisée en ce qu_{'}elle comprend de 10 à 26 parties en poids de ginseng et de 5 et 13 parties en poids de réglisse.

3. Composition pharmaceutique à utiliser selon la revendication 1, **caractérisée en ce qu'**elle comprend du jujube.

4. Composition pharmaceutique à utiliser selon la revendication 3, **caractérisée en ce qu'**elle comprend de 4 à 58 parties en poids de ginseng, de 2 à 28 parties en poids de réglisse et de 2 à 38 parties en poids de jujube, de préférence **caractérisée en ce qu'**elle comprend de 10 à 26 parties en poids de ginseng, de 5 à 13 parties en poids de réglisse et de 4 à 16 parties en poids de jujube.

5. Composition pharmaceutique à utiliser selon la revendication 3, **caractérisée en ce que** le jujube est extrait pour obtenir un premier extrait qui a une première concentration en AMPc de jujube, le premier extrait étant de plus extrait pour obtenir un deuxième extrait qui a une deuxième concentration en jujube, la deuxième concentration en AMPc de jujube étant supérieure à la première concentration en AMPc de jujube.

6. Composition pharmaceutique à utiliser pour traiter des troubles d'anxiété, **caractérisée en ce qu'**elle comprend un ginsenoside qui comporte un Rg 1 et un Rb 1 et un acide lié de manière glycyrrhizique choisi parmi le groupe qui comprend l'acide glycyrrhizique, l'acide glycyrrhétinique et leur combinaison.

7. Composition pharmaceutique à utiliser selon la revendication 6, **caractérisée en ce qu'**elle comprend de 2 à 24 parties en poids de ginsenoside et de 3 à 45 parties en poids d'acide lié de manière glycyrrhizique, de préférence **caractérisée en ce qu'**elle comprend de 4 à 11 parties en poids de ginsenoside et de 5 à 14 parties en poids d'acide lié de manière glycyrrhizique

8. Composition pharmaceutique à utiliser selon la revendication 6, **caractérisée en ce qu'**elle comprend en outre une adénosine monophosphate cyclique de jujube (AMPc de jujube).

9. Composition pharmaceutique à utiliser selon la revendication 8, **caractérisée en ce qu'**elle comprend de 2 à 24 parties en poids de ginsenoside, de 3 à 45 parties en poids d'acide lié de manière glycyrrhizique et de 0,003 à 0,4 partie en poids d'AMPc de jujube, de préférence **caractérisée en ce qu'**elle comprend de 4 à 11 parties en poids de ginsenoside, de 5 à 14 parties en poids d' acide lié de manière glycyrrhizique et de 0,01 à 0,07 partie en poids d'AMPc de jujube.

10. Composition pharmaceutique à utiliser selon la revendication 6, **caractérisée en ce que** le ginsenoside est extrait du ginseng, l'acide lié de manière glycyrrhizique est extrait de la réglisse et l'AMPc de jujube est extrait de jujube.

11. Composition pharmaceutique à utiliser selon la revendication 6, en outre **caractérisée en ce qu'**elle comprend au moins soit un vecteur acceptable en pharmacologie soit un additif qui est produit sous forme d'aliment diététique ou d'un complément et dont la forme posologique est choisie parmi le groupe formé par le comprimé, la gélule, la poudre, la pilule, la poudre fine, la solution, la microgélule, la suspension, l'émulsion, la molécule, les gouttes et le rouleau.

12. Procédé de préparation pour une adénosine monophosphate cyclique de jujube (AMPc de jujube) dans une composition pharmaceutique à utiliser pour traiter des troubles d'anxiété, **caractérisé par** les étapes consistant à
(a) procéder à l'extraction du jujube pour obtenir un premier extrait qui présente la première concentration en AMPc de jujube,
(b) purifier le premier extrait en chromatographiant le premier extrait par une résine microporeuse liée à un groupe aldéhyde pour obtenir un deuxième extrait qui présente une deuxième concentration en AMPc de jujube,
la deuxième concentration en AMPc de jujube étant supérieure à la première concentration en AMPc de jujube.
